# EUROPEAN PATENT SPECIFICATION

(11) **EP 2 165 681 B1**
(45) Date of publication and mention of the grant of the patent: **31.07.2013**
(21) Application number: 09012037.9
(22) Date of filing: 22.09.2009
(51) Int. Cl.: A61F 2/30, A61F 2/38, A61F 2/42, A61F 2/00

(54) **Fixed bearing joint endoprosthesis with combined congruent - incongruent prosthetic articulations**
sowohl kongruent als auch inkongruent gelenkig gelagerte Endoprothese mit stationärem Lager
Endoprothèse à roulement fixe avec des articulations prosthétiques congruentes/incongruentes combinées

(30) Priority: 22.09.2008 US 98824 P
(43) Date of publication of application: 24.03.2010
(73) Proprietor: Buechel-Pappas Trust, South Orange, New Jersey 07079 (US)
(72) Inventor: Pappas, Michael J., Caldwell, New Jersey 07006 (US)
(74) Representative: Müller-Boré & Partner Patentanwälte

(56) References cited:
- WO-A2-2007/119173
- GB-A- 2 253 147
- US-A- 5 871 539

## Description

### BACKGROUND OF THE INVENTION

1. Field of the Invention. The invention relates to prosthetic joints, such as prosthetic knee joints.

2. Description of the Related Art. A typical prosthetic knee includes a tibial component for mounting to the resected proximal end of the tibia, a femoral component for mounting to the resected distal end of the femur, a bearing between the tibial and femoral components and a patellar component mounted to the posterior face of the patella. The tibial and femoral components typically are made of metal and the bearing typically is made of plastic, such as UHMWPe. The proximal or superior surface of the bearing is formed to define medial and lateral concave regions. The distal or inferior surface of the femoral component is formed to define medial and lateral convex condyles that articulate in bearing engagement with the concave regions of the bearing. Some prosthetic knees include a mobile bearing that is permitted to undergo controlled rotational and translational movement relative to the tibial component. Other prosthetic knees include a bearing that is fixed relative to the tibial component.

Knee motion is highly complex and includes flexion-extension, axial rotation, anterior-posterior translation, and adduction-abduction. Incongruency between the femoral component and the bearing enables these complex motions to be carried out with enhanced mobility for the patient who has a prosthetic knee joint Accordingly, many prosthetic knee joints provide highly incongruent contact between the femoral component and the bearing. Incongruent contact causes a specified load to be applied to a small area, and hence causes the contact stress (load per unit area) to be higher than in a knee joint with more congruent contact. The metallic and plastic materials currently used in joint replacement permit normal knee motion with contact stresses that can accommodate normal physiological loads over an extended period of time in mobile bearing prosthetic knees. For example, U.S. Patents 4,309,778 and 4,340,978 disclose mobile bearing prosthetic knee joints with tibiofemoral articulation surfaces that have demonstrated an ability to last for an extended time.

Incongruent contact is particularly important in fixed bearing designs in view of the complex combinations of flexion-extension, axial rotation, anterior-posterior translation, and adduction-abduction associated with knee motion. However, fixed bearing prosthetic knee joints can produce contact stresses greatly in excess of acceptable limits associated with the strength of UHMWPe normally used for the tibial articulation surface. The dilemma for designers of fixed bearing knees is to effect a compromise between the conflicting requirements for joint motion mobility (which is accomplished by increasing contact surface incongruity and thus contact stress) and low contact stress (which requires high congruity and thus low joint mobility) to prevent rapid failure of the plastic used in current prosthetic joint articulations. Unfortunately a satisfactory compromise has yet to be found where fixed bearing knee components can be considered safe for extended use under normal physiological loads. A similar situation is true for other load bearing condylar joints such as the tibiotalar ankle joint.

The United States Food and Drug Administration (USFDA) requires extensive and rigorous clinical testing before approval of most mobile bearing joint replacements, and hence inhibits the use of such devices. The USFDA does not require similar testing for fixed bearing devices. Thus, most knee devices and all ankle devices that are generally available in the United States are the lower performing fixed bearing devices.
WO 2007/119173 discloses a knee prosthesis comprising a femoral component and a tibial component. The femoral component comprises a medial femoral condyle having a medial femoral condylar surface and a lateral femoral condyle having a lateral femoral condylar surface. The tibial component comprises a medial tibial condyle having a medial tibial condylar surface and a lateral tibial condyle having a lateral tibial condylar surface. The medial femoral condylar surface comprises a part-spherical convex surface and the medial tibial condylar surface comprises a part-spherical concave surface, the part-spherical surfaces being arranged to enable the medial femoral condyle to engage in sphere-in-sphere engagement with the medial tibial condyle. The sphere-in-sphere engagement provides anterior-posterior stability of the femoral component relative to the tibial component.; The lateral tibial condylar surface comprises a track surface for the lateral femoral condyle to move across as the medial condyle pivots around the sphere-in-sphere engagement. The track surface is posteriorly unrestricted to permit the lateral femoral condylar surface to contact the track surface at a range of contact positions as the medial femoral condyle pivots relative to the medial tibial condyle around the sphere-in-sphere engagement.
GB 2 253 147 A discloses a knee prosthesis comprising a femoral component having a medial condyle and a lateral condyle and a tibial component. The rolling surface of the medial condyle is part-spherical and the tibial component has a complementary part-spherical depression in its upper surface. The bearing surface of the lateral condyle includes a posterior part which has a curvature in a substantially sagittal plane about a first point on a transverse axis that passes through the centre of curvature of the rolling surface of the medial condyle and an anterior part which has a curvature about a point that lies on a second transverse axis parallel to, and anterior to, the first transverse axis.; The tibial component has an arcuate groove to receive the lateral condyle and to permit, in flexion of the knee after implantation, limited anterior/posterior movement of the lateral side. In flexion of the knee the posterior part of the rolling surface of the lateral condyle is received in this arcuate groove but, as the knee straightens and approaches its straightened condition, the anterior part of this rolling surface bears on the anterior end of the groove and thereby forces the lateral side of the tibial component to move anteriorly relative to the lateral condyle by a camming action. In this way anterior/ posterior movement of the lateral side of the tibia relative to the femur is hindered in the straightened condition of the knee.

### SUMMARY OF THE INVENTION

Improved fixed bearing articulating surfaces are possible by limiting the degree of incongruity in such devices. This may be accomplished by using a congruent, spherical surface on the medial condyle of the knee or ankle and mildly incongruent line contact on the more lightly loaded lateral condyle rather than the typical point contact on both sides used for fixed bearing designs. This design recognizes the fact that the medial condyles of both the femur and the patella of the knee joint and the medial condyle of the ankle joint are subject to greater loads than the lateral condyles thereof. The congruent contact at the more highly loaded medial condyle results in lower stress (i.e. force per unit area) due to the higher surface contact area achieved with congruency. On the other hand, the line contact at the less highly loaded lateral condyle results in acceptably low stress despite the smaller surface area due to the lower load on the lateral condyle. However, the line contact at the lateral condyles can achieve greater joint mobility without using a mobile bearing joint design.

Such a surface can be designed to accept normal walking loads within the allowable stress limits of the materials used in such joint replacement while still providing needed joint mobility. Expected stresses on the lateral condyle will, however, be substantially greater than that of a comparable mobile bearing with congruity on both sides. The combined congruenent-incongreuent articulating surface is thus an acceptable, although less desirable, design compromise to accommodate the regulatory requirements of the USFDA and the many surgeons who have become accustomed to fixed bearings.

Many patients who receive knee and ankle implants are quite elderly and inactive and thus produce loads that are substantially less than normal. This lower loading level (producing lower contact stresses for a given articulation geometry), coupled with the reduced time and frequency of use (which reduce the accumulated damage for given contact stresses) can allow articulating surfaces with a greater degree of incongruity and thus allow the use of fixed bearing components. Since fixed bearings do not require a supporting prosthetic platform, they can be fixtured directly to bone, saving the cost of the platform. The US medical care system is under considerable pressure to lower costs, and hence many hospitals would prefer to use a low cost device. A low cost, fixed bearing, device can be used as tibial or patellar components of a total knee in an elderly, inactive, patient. Therefore, the added cost of multi-part tibial or patellar replacements are not justified economically if a lower cost set of components are adequate.

An articulation surface with partially incongruent contact surfaces can produce substantially lower contact stresses than existing incongruent, fixed bearing devices. Lowering contact stresses in incongruent fixed bearing devices reduces wear and fatigue damage of the prosthetic articulating surfaces, thereby increasing their service life and increasing the population group to which such components can safely be used. The articulating surfaces of the subject invention can have similarities to the articulating surfaces shown in U.S. Patent No. 5,871,539 and U.S. Patent No. 6,074,425. However, the articulating surfaces of the subject invention are formed by means that are different from the means used to generate the articulating surfaces in these earlier patents. Additionally, the articulating surfaces of the subject invention are configured to achieve line contact in only one of the condyles of the subject invention as compared to both condyles of the earlier patents. Thus, this invention improves the fixed bearing articulating surfaces.

### BRIEF DESCRIPTION OF THE DRAWINGS

Fig. 1 side elevational view of a knee that has a total knee replacement prosthesis in accordance with one embodiment of the invention.

Fig. 2 is a front elevational view of the knee and prosthesis of Fig. 1.

Fig. 3 is of the assembled components of the prosthesis of Figs 1 and 2 independent of the knee.

Fig. 4 is a front elevational view of the prosthesis of Fig. 3

Fig. 5 is a top plan view of the tibial articular surface of the knee prosthesis of Figs 3 and 4.

Fig. 6 is a front elevational view of a blank for forming the tibial component of the prosthesis and a cutter for forming the articular surface on the blank.

Fig. 7 is an exploded side elevational view of the blank for forming the tibial component of the prosthesis and the cutter of Fig. 6.

Fig. 8 is a front elevation view of the tibial component and the cutter near the completion of a cutting operation.

Fig. 9 is a side elevational view of the tibial component and the cutter in the relative positions shown in Fig. 8.

Fig. 10 is a cross sectional view of the tibial component of the knee prosthesis formed by the cutter as taken along an anterior-posterior line through the lateral condylar surface.

Fig. 11 is a side elevational view of the tibial and femoral components assembled and articulated relative to one another.

Fig. 12 is a front elevational view of the femoral component and the patellar component of the prosthesis.

Fig. 13 is a top plan view, partly in section, shown the assembled knee prosthesis at full extension.

Fig. 14 is a front elevational view, partly in section, of an ankle prosthesis in accordance with the invention.

Fig. 15 is a side elevational view of the ankle prosthesis of Fig. 14.

Fig. 16 is a cross sectional view of the tibial component of the ankle prosthesis of Figs 14 and 15.

Fig. 17 is a bottom plan view of the ankle prosthesis of Figs. 14 and 15.

Fig. 18 is a front elevational view of the bearing of the ankle prosthesis of Figs. 14 and 15.

Fig. 19 is a side elevational view of the bearing shown in Fig 18.

Fig. 20 is a cross-sectional view taken along line A-A of Fig. 17.

Fig. 21 is a cross-sectional view taken along line B-B of Fig. 17.

Fig. 22 is a bottom plan view of the bearing.

Fig. 23 is a cross-sectional view taken along line C-C of Fig. 22.

Fig. 24 is a front elevational view of the talar component of the ankle prosthesis of Figs 14 and 15.

Fig. 25 is a side elevation component of the talar component of Fig. 24.

Fig. 26 is a side elevational view of a knee prosthesis in accordance with a third embodiment of the invention.

Fig. 27 is a front elevational view of the knee prosthesis of Fig. 26.

Fig. 28 is a front elevational view similar to Fig. 27, but showing the bearing and the tibial component in section along a medial-lateral line.

Fig. 29 is a top plan view of the bearing and the tibial component.

Fig. 30 is a top plan view of the tibial component.

Fig. 31 is a bottom plan view of the bearing.

### DETAILED DESCRIPTION OF THE PREFERRED EMBODIMENTS

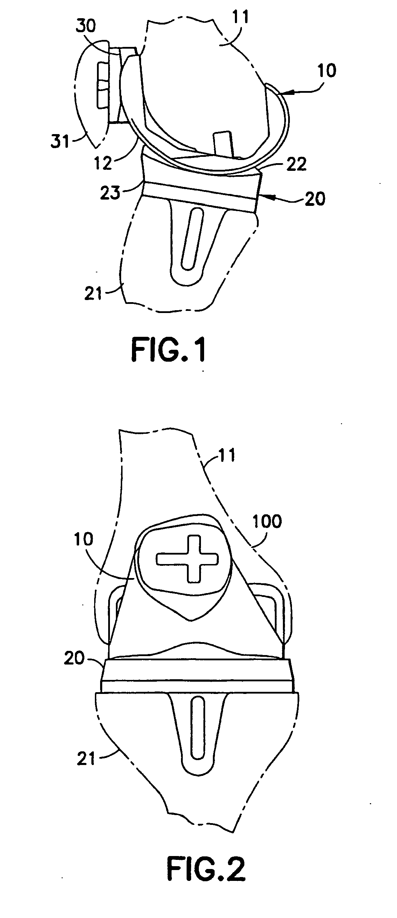

Figs. 1 and 2 show a cruciate sacrificing total knee replacement prosthesis 100. The knee prosthesis has a metallic (Co-Cr or Titanium alloy) femoral component 10 which is fixtured to the distal femur 11, a plastic (UHMWPe) tibial component 20 fixtured to the proximal tibia 21, and a plastic patellar component 30 fixtured to the posterior patella 31. Alternately, both components may be metallic, ceramic coated metal or ceramic.

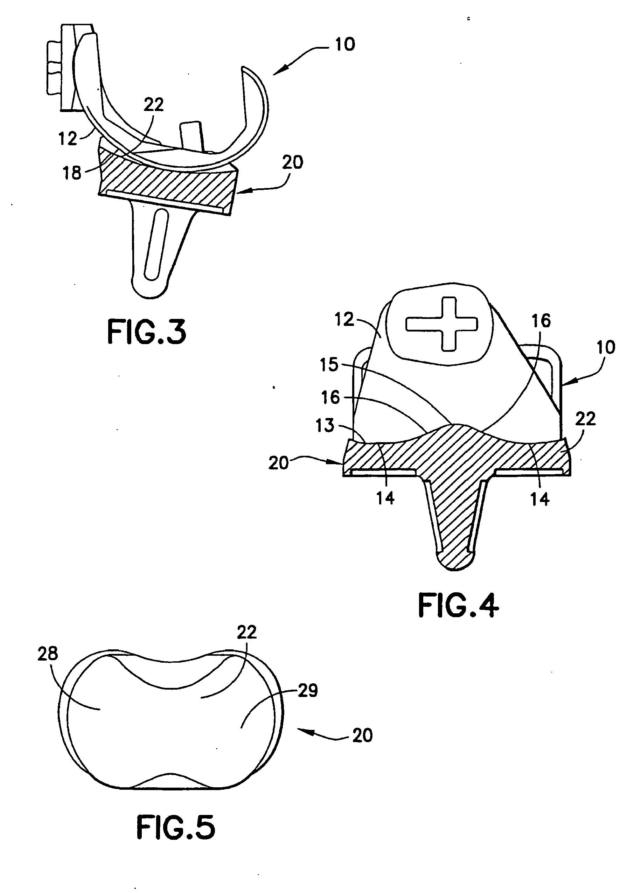

The geometry of the femoral articulating surface 12 of the femoral component 10, as shown in Figs. 3 and 4, is a compound surface of revolution generated by revolving a generating curve 13 consisting of radii 14, radius 15, and connecting tangents 16. This geometry is described in additional detail in the above-referenced patents, including U.S. Patent No. 4,309,778 and U.S. Patent No. 5,507,820.

The tibial component 20 has a tibial articulating surface 22 that is generated using the same generating curve 13, except for different connecting tangents. However, only the medial articulation 28 of the tibial articulating surface 22 is a surface of revolution, and the lateral surface 29 is not a surface of revolution. Rather, the lateral tibial articulating surface 29 is generated by simultaneously rotating a surface of revolution about different axes. This generation method is unique and useful. The surface of revolution for the medial articulation 28 of the tibial articulating surface 22 preferably is configured relative to the compound surface of revolution of the femoral articulating surface 12 to achieve congruency to at least about 40-50 degrees of flexion. Line contact may exist between the femoral component 10 and the tibial component 20 at greater flexion.

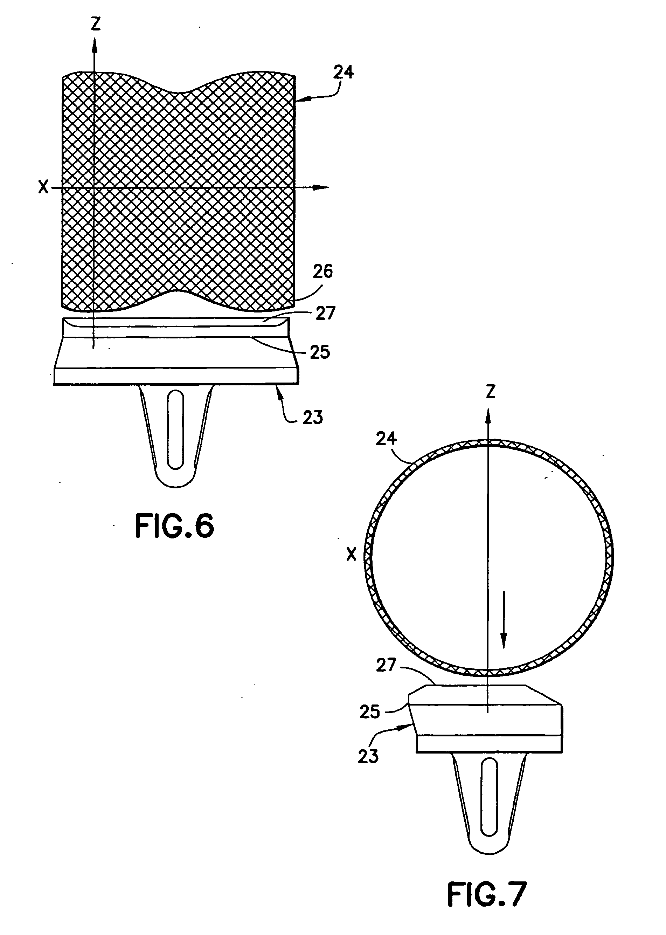

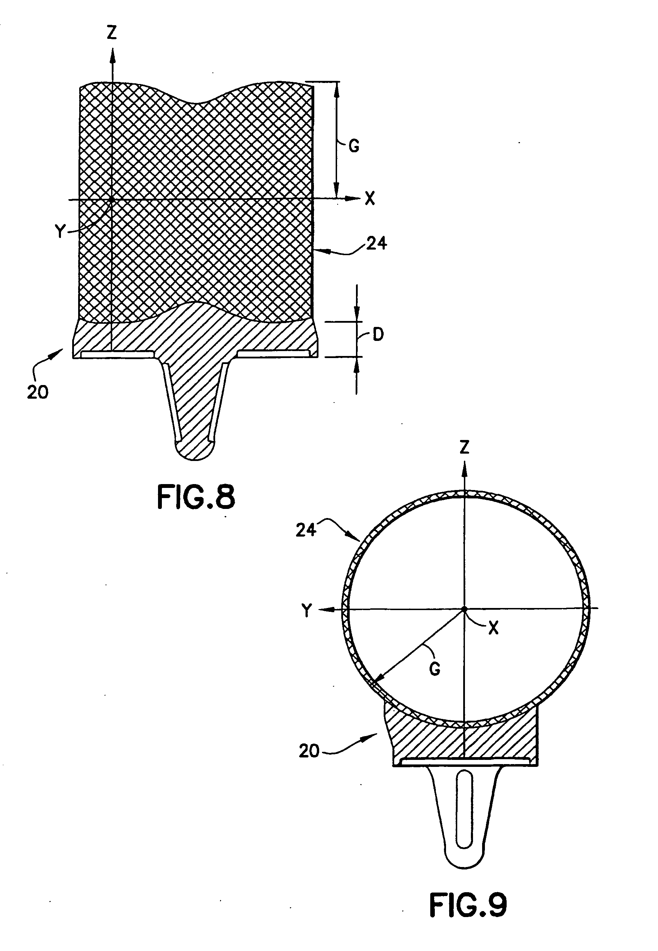

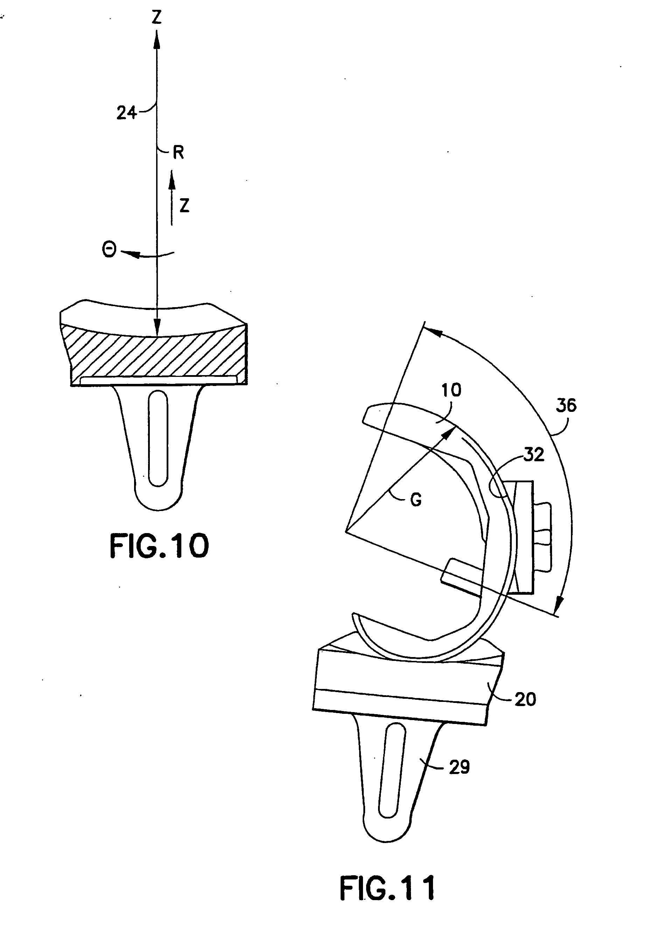

The tibial articulating surface 22 may be formed on a tibial component blank 23, as shown in Figs. 6 and 7, by a cutter 24 made in the form of a surface of revolution formed by the generating curve 13 shown in Fig. 4. The cutter 24 initially is rotated about axis X-X, fixed in the cutter, as shown in Fig. 6. The axis X-X is parallel to the face 25 of tibial component blank 23 from a position where axis X-X of the cutter 24 intersects the Z axis. In this initial position, the cutting surface 26 of the cutter 24 is above the top surface 27 of tibial component blank 23, as shown in Figs. 6 and 7. The cutter 24 then is moved along the Z axis into the blank 23 until the cutter 24 has cut the blank 23 to the desired depth D as shown in Fig. 8. From this position the cutter 24 simultaneously is rotated about the Y and Z axes, as shown in Fig. 9 to create a lateral condylar surface 29 with principal radii R and G at the line of lateral contact where R is larger than radius G, as shown in Figs 10 and 11. This manufacturing method results in an articulating surface 22 that is congruent to the femoral surface 12 on the medial condyle and in line contact on the lateral condyle under compressive loading of the joint 100 during axial rotation of the tibia 21 relative to the femur 11. The desired size of the radius R compared to the radius G is dependent on the degree of axial rotation needed in normal joint motion. An increase in radius R decreases valgus-varus tibial rotation about the Y axis during axial (Z axis) rotation and increases the amount of axial rotation before line contact is lost on the lateral articulating surfaces. Unfortunately increasing radius R also increases the degree of incongruity.

This resulting surface will be referred to here as a "medial-pivot" surface since motion on the medial articulation of the tibia 21 relative to the femur will take place about the origin of the X, Y and Z axes, fixed to the tibia with the X, Y, Z coordinate system origin at the center of the spherical medial articulating surfaces.

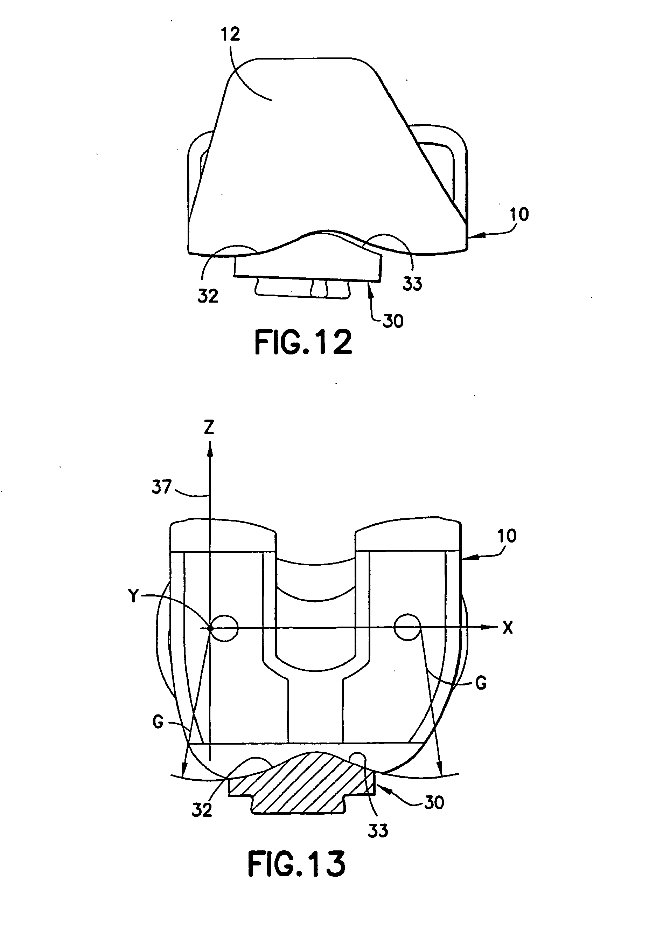

Loads that press the patellar component 30 to the femoral component articulating surface 12 are low at full extension. However, at about 35-45 degrees flexion, the substantial load caused by the quadriceps pulls the patellar component 30 medially into the sulcus. Thus, the medial patellar articulation surface 32 carries most of the load, Often the lateral patellar articulating surface 33 lifts off the femoral component articulating surface 12, as shown in Fig. 12. Where this occurs a medial-pivot surface will produce congruent contact on the medial articulation 32 and since the contacting surfaces are spherical it allows rotation about three independent axes under congruent contact.

Where the medial component of the patellofemoral compressive load is sufficient so as not to produce lift off of lateral patellar articulation surface 33, as shown in Fig. 11, congruent articulation at the medial patellar articulation surface 32 will still occur but articulation at the lateral patellar articulation surface 33 will be incongruent. The normal axial rotation of the patella 31 is less than associated with the tibiofemoral articulation. Thus, somewhat smaller radius R may be used to reduce the degree of incongruity, thereby reducing the lateral surface contact stress.

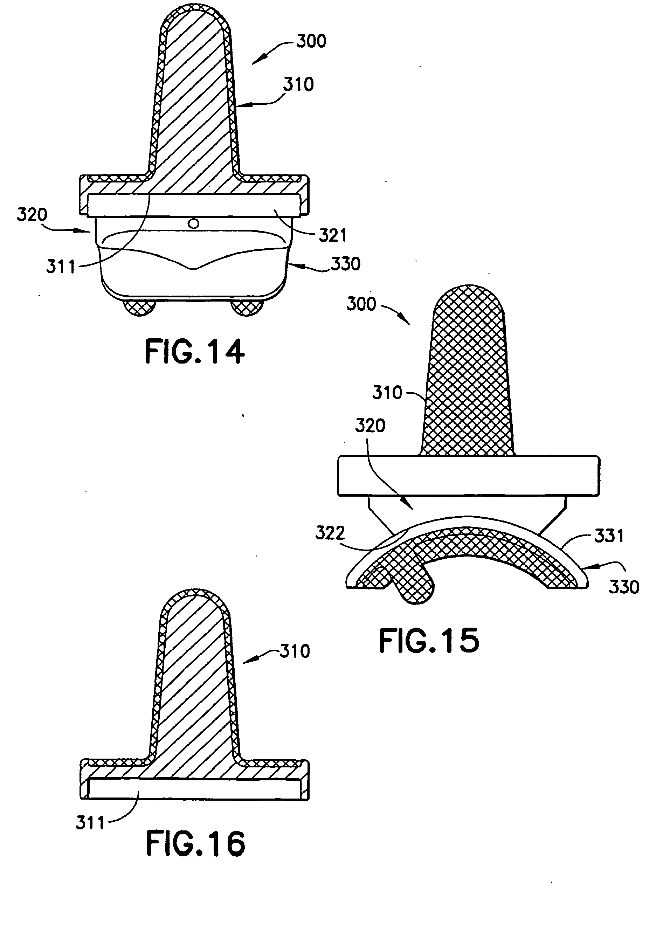

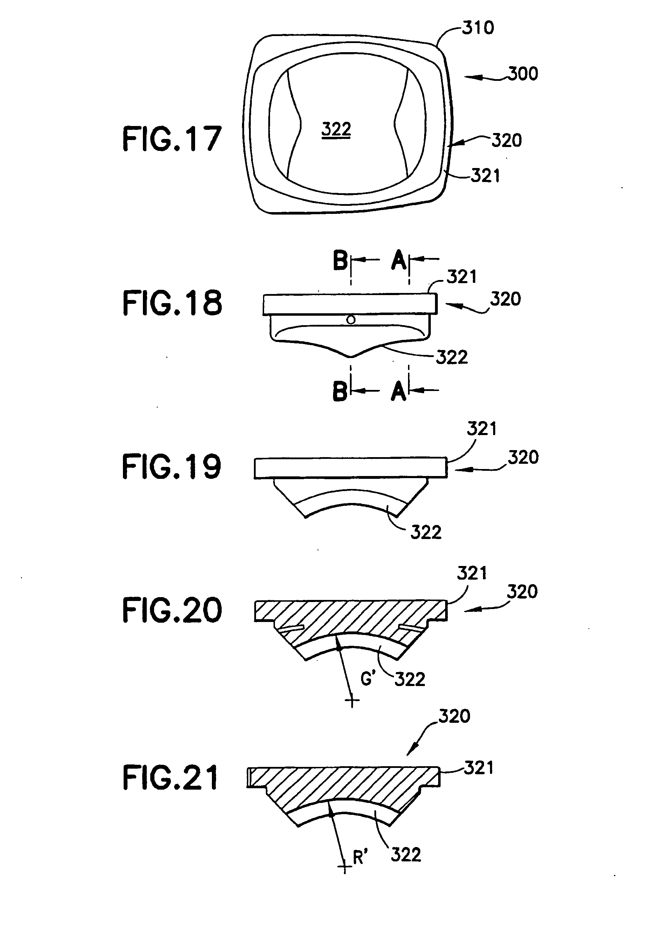

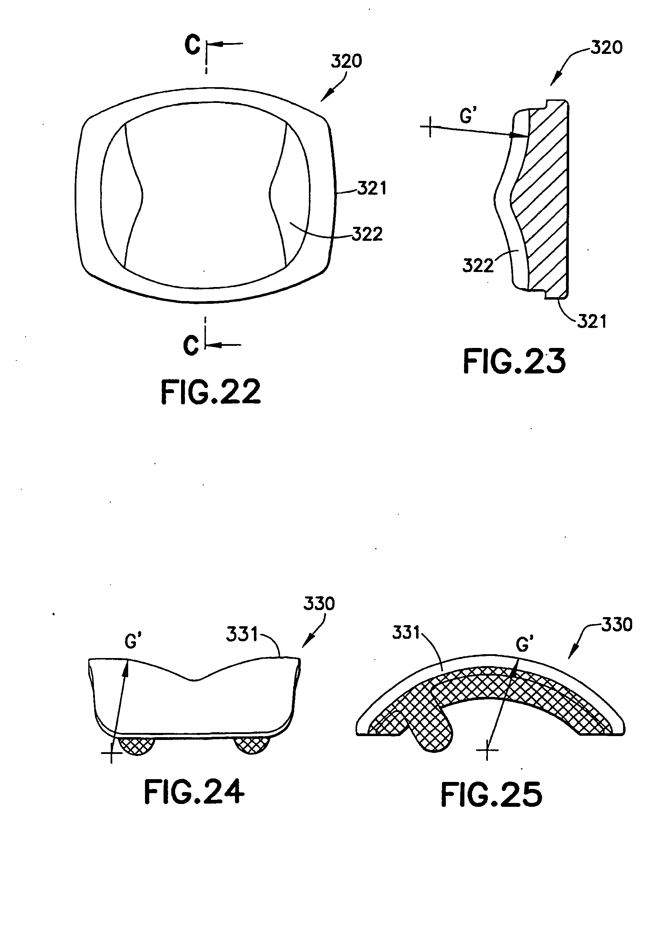

Figs 14-25 illustrate an ankle prosthesis 300 in accordance with the invention. The ankle prosthesis 300 has a tibial component 310, a bearing 320 and a talar component 330. The bearing 320 has a plate 321 that fits snuggly into cavity 311 of the talar component 310 to prevent movement of the bearing relative to the talar component under compressive load. This arrangement causes the bearing 320 to be considered a "fixed" bearing. The bearing 320 also has a bearing articulating surface 322 of bearing that articulates with a talar articulating surface 331 of the talar component 330. The talar articulating surface 331 of the talar component 330 is a surface of revolution generated by rotating a generating curve similar in shape to 13, except reduced in scale. The bearing articulating surface 322 of the bearing 320 is generated in exactly the same fashion as the knee tibial articulating surface 22. However, axial rotation of the ankle is small compared to the knee. Therefore, a radius R' may be much closer in size, proportionately, to the radius G' than the radius R is to the radius G. Thus, the increase in contact stress due to the introduction of incongruity is substantially less in the ankle than in the knee. Such reduction is needed because contact stresses in the ankle, even for congruent contact, are substantially greater than in the knee due to the fact that, although loads in the knee and ankle are similar, the ankle is much smaller than the knee.

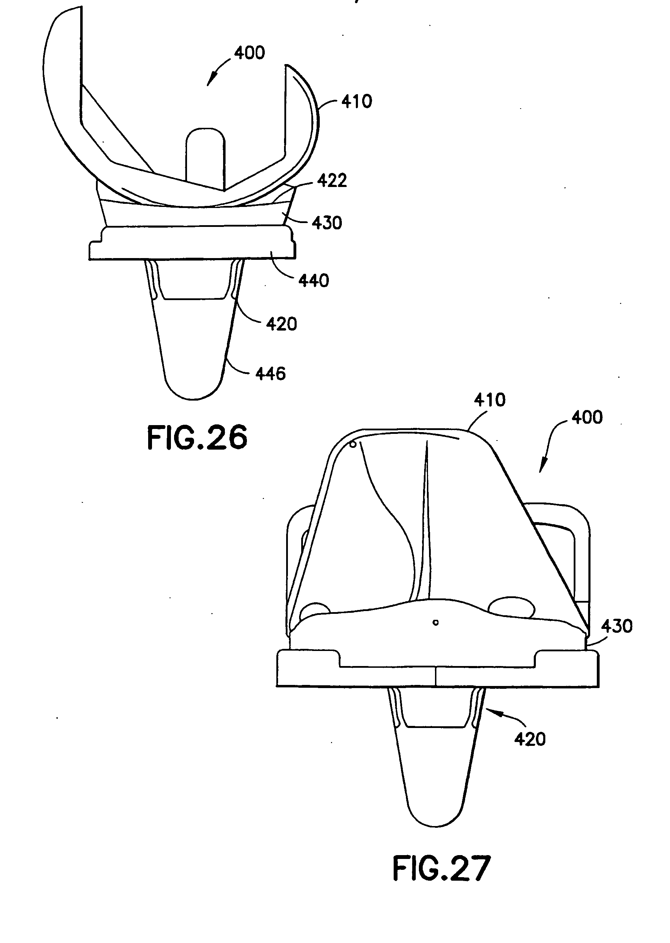

A replacement knee in accordance with a third embodiment is identified by the numeral 400 in Figs 26-31. The replacement knee 400 has a femoral component 410 and tibial articulating surface 422 that are the same as in the replacement knee 100 of the first embodiment. However, the replacement knee 400 differs from the replacement knee 100 in that the tibial component 420 of the replacement knee 400 comprises two parts, namely, a bearing 430, made of a plastic such as UHMWPe and a metallic 440 tray, made of Co-Cr or Titanium alloy.

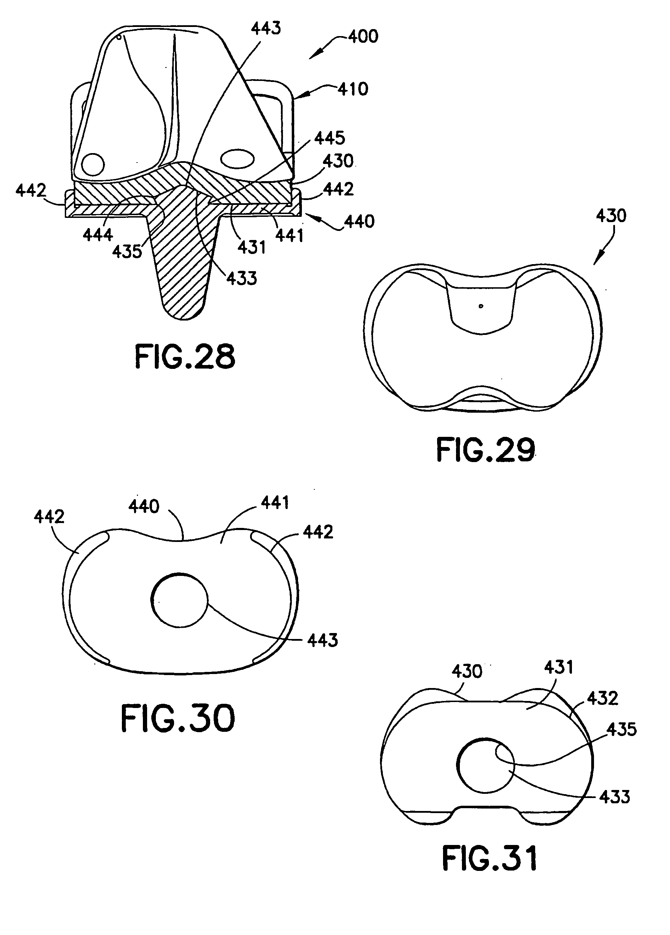

Referring to Fig. 30, the tray 440 has a platform 441 with two vertical walls 442. A button 443 projects up from the platform 441, as shown in Fig. 28. The button 443 is formed with a ridge 444 and an undercut 445. Fixation surfaces 446 are defined on a lower or inferior part of the tray 440, as shown in Fig. 26. Referring to Fig. 31 the bearing 430 has a flat inferior surface 431 and side surfaces 432 extend up from the inferior surface 431. A hole 433 extends into the inferior surface 431 and is formed with a ridge 435, as shown in Fig. 28. The bearing 430 is assembled onto the tray 440 by placing the hole 433 on the button 443 and pushing the bearing toward the tray 440, while aligning the tray sidewalls 442 with the bearing side surface 432 until the ridge 435 of the hole 433 expands over the ridge 444 of the button 443 and the bearing 430 snaps into place, as shown in Fig. 28. The dimensions of the side surfaces 432 of the bearing 430 and the sidewalls 442 of the tray 440 are selected to produce a close slip, to light press fit so as to minimize any motion between the bearing 430 and the tray 440.

The medial-pivot surface need not be formed by use of a cutter such cutter 34, which is used primarily for purposes of illustration. A medial-pivot surface can be machined by a variety of cutters including form cutters, point cutters, and ball mills using two and three dimensional computer driven machines.

A medial-pivot surface is unique within and without the field of orthopedic surgical appliances. In human replacement joints its primary application is in condylar joints such as the knee, ankle great toe, pip joint of the finger, and the thumb and in the elbow.

## Claims

1. An orthopedic prosthetic joint replacement (100, 300, 400) comprising: a first joint component (10, 310, 410) having medial and lateral convex condyles formed with spherical condylar segments defining first and second radii respectively, and a second joint (20, 320) component having a medial concave spherical condylar segment (28) with a radius equal to the radius of the medial convex spherical condylar segment, the second joint component (20, 320) further having a lateral concave non-spherical condylar segment (29), the medial convex spherical condylar segment of the first joint component (10, 310, 410) being in congruent contact with the medial concave spherical condylar segment (28) of the second joint component (20, 320), the lateral convex spherical condylar segment of the first joint component (10, 310, 410) being in line contact with the lateral non-spherical concave condylar segment (29) of the second joint component (20, 320) during a loading that presses the components together.

2. The orthopedic prosthetic joint replacement (100, 300, 400) of claim 1, wherein the first joint component (10, 310, 410) is formed from metal and wherein at least the medial and lateral concave condylar segments (28, 29) of the second joint component (20, 320) are formed from a non-metallic material.

3. The orthopedic prosthetic joint replacement (100, 300, 400) of claim 1 or 2, wherein the second joint component (20, 320) includes a metallic component having a fixation surface for fixed mounting to a bone and a non-metallic bearing engaged with the metallic component, the medial and lateral concave condylar segments (28, 29) being formed on the bearing.

4. The orthopedic prosthetic joint replacement (100, 300, 400) of claim 3, wherein the bearing of the second joint component (20, 320) is fixed relative to the metallic component of the second joint component (20, 320).

5. The orthopedic prosthetic joint replacement (100, 300, 400) of any of claims 1-4, wherein the convex and concave medial condylar segments are spherical and have substantially equal radii.

6. The orthopedic prosthetic joint replacement (100, 300, 400) of any claims 1-5, further comprising a third joint component (30) having concave medial and lateral condylar segments (32, 33), the concave medial condylar segment (32) of the third joint component (30) being configured for congruent articular bearing engagement with the convex medial condylar segment of the first joint component (10, 310, 410), the concave lateral condylar segment (33) of the third joint component (30) being configured for incongruent line contact with the convex lateral condylar segment of the first joint component (10, 310, 410) during articulation of the prosthetic joint.

7. The orthopedic prosthetic joint replacement (100, 300, 400) of claim 6, wherein the first joint component (10, 310, 410) is a femoral component with a superior surface configured for fixation to a femur (11), the second joint component (20, 320) is a bearing fixed to a tibial component that has an inferior surface configured for fixation to a tibia (21) and the third joint component (30) is a patellar component with an anterior surface configured for fixation to a patella (31).

8. The orthopedic prosthetic joint replacement (100, 300, 400) of claim 6 or 7, wherein the convex condyles of the first joint component (10, 310, 410) are formed from metal and the concave condyles of the second and third joint components (20, 320, 30) are formed from plastic.

9. The orthopedic prosthetic joint replacement (100, 300, 400) according to any of claims 1-6, wherein the joint replacement (100, 300, 400) is a ti-biofemoral knee replacement (100, 400).

10. The orthopedic prosthetic joint replacement (100, 300, 400) according to any of claims 1-6, wherein the joint replacement (100, 300, 400) is a tibiotalar ankle replacement (300).

11. The orthopedic prosthetic joint of remplacement (100, 300, 400) according to any of claims 1-6, wherein the joint replacement (100, 300, 400) is a prosthetic replacement joint for a joint in a hand.

12. The orthopedic prosthetic joint replacement (100, 300, 400) according to any of claims 1-6, wherein the joint is a prosthetic replacement joint (100, 300, 400) for a joint in an elbow.

13. A method for forming the orthopedic prosthetic joint replacement (100, 300, 400) of any one of claims 1-12, the method comprising forming a locus of points defined by the concave condylar segments (28, 29), comprising the steps of:
providing a cutter (24) with a first axis (X) and a cutting surface (26) defined concentrically around the first axis (X), the cutting surface (26) having medial and lateral convex cutting areas for forming the medial and lateral concave condylar segments (28, 29);
rotating the cutter (24) around the first axis (X);
advancing the cutter (24) toward a blank (23) along a second axis (Z) that is substantially perpendicular to the first axis (X), while continuing to rotate the cutter (24) around the first axis (X); and
pivoting the cutter (24) around a third axis (Y) that is substantially perpendicular to the first and second axes (X, Z) while continuing to rotate cutter (24) around the first axis (X).

14. The method of claim 13, wherein the locus of points further is formed by pivoting the cutter (24) around the second axis (Z) simultaneously with the step of pivoting the cutter (24) around the third axis (Y).

## Patentansprüche

1. Orthopädische Gelenkersatzprothese (100, 300, 400) umfassend:
eine erste Gelenkkomponente (10, 310, 410), die mediale und laterale, konvexe Kondylen aufweist, die aus kugelförmigen, kondylären Segmenten gebildet sind, die jeweils erste und zweite Radien bestimmen, und eine zweite Gelenkkomponente (20, 320), die ein mediales, konkaves, kugelförmiges, kondyläres Segment (28) mit einem Radius aufweist, der gleich dem Radius des medialen, konvexen, kugelförmigen, kondylären Segments ist, wobei die zweite Gelenkkomponente (20, 320) überdies ein laterales, konkaves, nichtkugelförmiges, kondyläres Segment (29) aufweist, wobei das mediale, konvexe, kugelförmige, kondyläre Segment der ersten Gelenkkomponente (10, 310, 410) in kongruentem Kontakt mit dem medialen, konkaven, kugelförmigen, kondylären Segment (28) der zweiten Gelenkkomponente (20, 320) ist, wobei das laterale, konvexe, kugelförmige Segment der ersten Gelenkkomponente (10, 310, 410) während einer Belastung, die die Komponenten aneinanderdrückt, in Linienkontakt mit dem lateralen, nicht-kugelförmigen, konkaven, kondylären Segment (29) der zweiten Gelenkkomponente (20, 320) ist.

2. Orthopädische Gelenkersatzprothese (100, 300, 400) nach Anspruch 1, wobei die erste Gelenkkomponente (10, 310, 410) aus Metall gebildet ist, und wobei mindestens die medialen und lateralen, konkaven, kondylären Segmente (28, 29) der zweiten Gelenkkomponente (20, 320) aus einem nichtmetallischen Material gebildet sind.

3. Orthopädische Gelenkersatzprothese (100, 300, 400) nach Anspruch 1 oder 2, wobei die zweite Gelenkkomponente (20, 320) eine metallische Komponente umfasst, die eine Befestigungsoberfläche für das feste Befestigen an einen Knochen aufweist, und ein nichtmetallisches Lager, das sich in Eingriff mit der metallischen Komponente befindet, wobei die medialen und lateralen, konkaven, kondylären Segmente (28, 29) auf dem Lager gebildet sind.

4. Orthopädische Gelenkersatzprothese (100, 300, 400) nach Anspruch 3, wobei das Lager der zweiten Gelenkkomponente (20, 320) bezogen auf die metallische Komponente der zweiten Gelenkkomponente (20, 320) stationär ist.

5. Orthopädische Gelenkersatzprothese (100, 300, 400) nach einem der Ansprüche 1 bis 4, wobei die konvexen und konkaven medialen kodylären Segmente kugelförmig sind und im Wesentlichen gleiche Radien aufweisen.

6. Orthopädische Gelenkersatzprothese (100, 300, 400) nach einem der Ansprüche 1 bis 5, überdies umfassend eine dritte Gelenkkomponente (30), die konkave, mediale und laterale, kondyläre Segmente (32, 33) aufweist, wobei das konkave, mediale, kondyläre Segment (32) der dritten Gelenkkomponente (30) ausgestaltet ist, in kongruentem, gelenkig gelagertem Eingriff mit dem konvexen, medialen, kondylären Segment der ersten Gelenkkomponente (10, 310, 410) zu sein, wobei das konkave, laterale, kondyläre Segment (33) der dritten Gelenkkomponente (30) während der gelenkigen Lagerung der Gelenkprothese für inkongruenten Linienkontakt mit dem konvexen, lateralen, kondylären Segment der ersten Gelenkkomponente (10, 310, 410) ausgestaltet ist.

7. Orthopädische Gelenkersatzprothese (100, 300, 400) nach Anspruch 6, wobei die erste Gelenkkomponente (10, 310, 410) eine femorale Komponente mit einer oberen Fläche ist, die für das Befestigen an einem Femur (11) ausgestaltet ist, wobei die zweite Gelenkkomponente (20, 320) ein Lager ist, das an einer tibialen Komponente befestigt ist, die eine untere Fläche aufweist, die für das Befestigen an einer Tibia (21) ausgestaltet ist, und die dritte Gelenkkomponente (30) eine patellare Komponente mit einer vorderen Fläche ist, die für das Befestigen an einer Patella (31) ausgestaltet ist.

8. Orthopädische Gelenkersatzprothese (100, 300, 400) nach Anspruch 6 oder 7, wobei die konvexen Kondylen der ersten Gelenkkomponente (10, 310, 410) aus Metall gebildet sind und die konkaven Kondylen der zweiten und dritten Gelenkkomponenten (20, 320, 30) aus Kunststoff gebildet sind.

9. Orthopädische Gelenkersatzprothese (100, 300, 400) nach einem der Ansprüche 1 bis 6, wobei der Gelenkersatz (100, 300, 400) ein tibiofemoraler Knieersatz (100, 400) ist.

10. Orthopädische Gelenkersatzprothese (100, 300, 400) nach einem der Ansprüche 1 bis 6, wobei der Gelenkersatz (100, 300, 400) ein tibiotalarer Fußgelenkersatz (300) ist.

11. Orthopädische Gelenkersatzprothese (100, 300, 400) nach einem der Ansprüche 1 bis 6, wobei der Gelenkersatz (100, 300, 400) eine Gelenkersatzprothese für ein Gelenk in einer Hand ist.

12. Orthopädische Gelenkersatzprothese (100, 300, 400) nach einem der Ansprüche 1 bis 6, wobei das Gelenk eine Gelenkersatzprothese (100, 300, 400) für ein Gelenk in einem Ellenbogen ist.

13. Verfahren zum Bilden der orthopädischen Gelenkersatzprothese (100, 300, 400) nach einem der Ansprüche 1 bis 12, wobei das Verfahren das Bilden eines geometrisches Ortes umfasst, der durch die konkaven, kondulären Segmente (28, 29) bestimmt wird, umfassend die folgenden Schritte:
Bereitstellen eines Schneidwerkzeugs (24) mit einer ersten Achse (X) und einer Schneidefläche (26), die konzentrisch um die erste Achse (X) bestimmt ist, wobei die Schneidefläche (26) mediale und laterale, konvexe Schneideflächen für das Bilden der medialen und lateralen, konkaven, kondylären Segmente (28, 29) aufweist;
Rotieren des Schneidwerkzeugs (24) um die erste Achse (X);
Vorwärtsbewegen des Schneidwerkzeugs (24) in Richtung eines Rohlings (23) entlang einer zweiten Achse (Z), die im Wesentlichen senkrecht zu der ersten Achse (X) ist, und dabei Fortsetzen des Rotierens des Schneidwerkzeugs (24) um die erste Achse (X); und
Schwenken des Schneidwerkzeugs (24) um eine dritte Achse (Y), die im Wesentlichen senkrecht zu der ersten und zweiten Achse (X, Z) ist, und dabei Fortsetzen des Rotierens des Schneidwerkzeugs (24) um die erste Achse (X).

14. Verfahren nach Anspruch 13, wobei der geometrische Ort überdies durch Schwenken des Schneidwerkzeugs (24) um die zweite Achse (Z) bei gleichzeitigem Schwenken des Schneidwerkzeugs (24) um die dritte Achse (Y) gebildet wird.

## Revendications

1. Remplacement articulaire prosthétique orthopédique (100, 300, 400) comprenant : un premier composant d'articulation (10, 310, 410) ayant des condyles convexes médial et latéral formés de segments condyliens sphériques définissant des premier et second rayons, respectivement, et un deuxième composant d'articulation (20, 320) ayant un segment condylien sphérique concave médial (28) avec un rayon égal au rayon du segment condylien sphérique convexe médial, le deuxième composant d'articulation (20, 320) ayant en outre un segment condylien non sphérique concave latéral (29), le segment condylien sphérique convexe médial du premier composant d'articulation (10, 310, 410) étant en contact congruent avec le segment condylien sphérique concave médial (28) du deuxième composant d'articulation (20, 320), le segment condylien sphérique convexe latéral du premier composant d'articulation (10, 310, 410) étant en contact linéaire avec le segment condylien non sphérique concave latéral (29) du deuxième composant d'articulation (20, 320) au cours d'un chargement qui presse les composants l'un contre l'autre.

2. Remplacement articulaire prosthétique orthopédique (100, 300, 400) selon la revendication 1, dans lequel le premier composant d'articulation (10, 310, 410) est formé de métal et dans lequel au moins les segments condyliens concaves médial et latéral (28, 29) du deuxième composant d'articulation (20, 320) sont formés d'un matériau non métallique.

3. Remplacement articulaire prosthétique orthopédique (100, 300, 400) selon la revendication 1 ou 2, dans lequel le deuxième composant d'articulation (20, 320) inclut un composant métallique ayant une surface de fixation pour le montage fixe à un os et un roulement non métallique engagé avec le composant métallique, les segments condyliens concaves médial et latéral (28, 29) étant formés sur le roulement.

4. Remplacement de prothèse articulaire orthopédique (100, 300, 400) selon la revendication 3, dans lequel le roulement du deuxième composant d'articulation (20, 320) est fixe par rapport au composant métallique du deuxième composant d'articulation (20, 320).

5. Remplacement articulaire prosthétique orthopédique (100, 300, 400) selon l'une quelconque des revendications 1 à 4, dans lequel les segments condyliens médiaux convexe et concave sont sphériques et ont des rayons essentiellement égaux.

6. Remplacement articulaire prosthétique orthopédique (100, 300, 400) selon l'une quelconque des revendications 1 à 5, comprenant en outre un troisième composant d'articulation (30) ayant des segments condyliens concaves médial et latéral (32, 33), le segment condylien concave médial (32) du troisième composant d'articulation (30) étant configuré pour un engagement de roulement articulaire congruent avec le segment condylien convexe médial du premier composant d'articulation (10, 310, 410), le segment condylien concave latéral (33) du troisième composant d'articulation (30) étant configuré pour un contact linéaire incongruent avec le segment condylien convexe latéral du premier composant d'articulation (10, 310, 410) au cours de l'articulation de l'articulation prosthétique.

7. Remplacement articulaire prosthétique orthopédique (100, 300, 400) selon la revendication 6, dans lequel le premier composant d'articulation (10, 310, 410) est un composant fémoral avec une surface supérieure configuré pour la fixation à un fémur (11), le deuxième composant d'articulation (20, 320) est un roulement fixé à un composant tibial qui a une surface inférieure configuré pour la fixation à un tibia (21) et le troisième composant d'articulation (30) est un composant patellaire avec une surface antérieure configurée pour la fixation à une rotule (31).

8. Remplacement articulaire prosthétique orthopédique (100, 300, 400) selon la revendication 6 ou 7, dans lequel les condyles convexes du premier composant d'articulation (10, 310, 410) sont formés de métal et les condyles concaves des deuxième et troisième composants d'articulation (20, 320, 30) sont formés de plastique.

9. Remplacement articulaire prosthétique orthopédique (100, 300, 400) selon l'une quelconque des revendications 1 à 6, dans lequel le remplacement articulaire (100, 300, 400) est un remplacement de genou tibio-fémoral (100, 400).

10. Remplacement articulaire prosthétique orthopédique (100, 300, 400) selon l'une quelconque des revendications 1 à 6, dans lequel le remplacement articulaire (100, 300, 400) est un remplacement de cheville tibio-talien (300).

11. Remplacement articulaire prosthétique orthopédique (100, 300, 400) selon l'une quelconque des revendications 1 à 6, dans lequel le remplacement articulaire (100, 300, 400) est une articulation de remplacement prosthétique pour une articulation de la main.

12. Remplacement articulaire prosthétique orthopédique (100, 300, 400) selon l'une quelconque des revendications 1 à 6, dans lequel l'articulation est une articulation de remplacement prosthétique (100, 300, 400) pour une articulation dans un coude.

13. Procédé de formation du remplacement articulaire prosthétique orthopédique (100, 300, 400) selon l'une quelconque des revendications 1 à 12, le procédé comprenant la formation d'un lieu géométrique défini par les segments condyliens concaves (28, 29), comprenant les étapes suivantes:
fournir un instrument tranchant (24) avec un premier axe (X) et une surface de découpe (26) définie de manière concentrique autour du premier axe (X), la surface de découpe (26) ayant des zones de découpe convexes médiale et latérale pour former les segments condyliens concaves médial et latéral (28, 29) ;
faire tourner l'instrument tranchant (24) autour du premier axe (X) ;
faire avancer l'instrument tranchant (24) vers une ébauche (23) le long d'un deuxième axe (Z) qui est essentiellement perpendiculaire au premier axe (X) tout en continuant de faire tourner l'instrument tranchant (24) autour du premier axe (X) ; et
faire pivoter l'instrument tranchant (24) autour d'un troisième axe (Y) qui est essentiellement perpendiculaire aux premier et deuxième axes (X, Z) tout en continuant de faire tourner l'instrument tranchant (24) autour du premier axe (X).

14. Procédé selon la revendication 13, dans lequel le lieu géométrique est en outre formé en faisant pivoter l'instrument tranchant (24) autour du deuxième axe (Z) simultanément à l'étape de pivotement de l'instrument tranchant (24) autour du troisième axe (Y).
